# EUROPEAN PATENT APPLICATION

(11) **EP 4 382 610 A1**
(43) Date of publication of application: **12.06.2024**
(21) Application number: 22876454.4
(22) Date of filing: 29.09.2022
(51) Int. Cl.: C12P 1/04, A61K 8/9728, A61K 8/9789, A61K 35/744, A61K 35/747, A61K 36/185, A61P 17/00, A61P 43/00, A61Q 19/02, C12N 1/20

(54) **ALTHAEA FERMENTED PRODUCT AND USE THEREOF**

(30) Priority: 01.10.2021 JP 2021162718
(71) Applicant: Kabushiki Kaisha Yakult Honsha, Tokyo, 105-8660 (JP)
(72) Inventor: KURASAWA, Tomoko, Tokyo 105-8660 (JP); SHIBATA, Shinya, Tokyo 105-8660 (JP); ITO, Masahiko, Tokyo 105-8660 (JP)
(74) Representative: Mewburn Ellis LLP
(86) International application number: PCT/JP2022/036454
(87) International publication number: WO 2023/054590

(57) **Abstract**

Provided is an Althaea-derived ingredient modified to increase activity.

A fermentation product having *Althaea* or a processed product thereof as a starting material, said fermentation product being obtained using one or more selected from microorganisms belonging to lactobacilli, microorganisms belonging to the genus Lactococcus, microorganisms belonging to the genus Leuconostoc, and microorganisms belonging to the genus Pediococcus. This fermentation product is useful as a topical skin agent, an ingredient blended into a cosmetic, and the like.

## Description

### TECHNICAL FIELD

The present invention relates to a plant fermentation product useful as a topical skin agent or the like, more specifically to an *Althaea* fermentation product and a topical skin agent containing the same.

### BACKGROUND ART

*Althaea* is a perennial plant native to Europe that grows in the wild on the coast of the Mediterranean Sea but is widely cultivated as an herb and for herbal medicines. Plant extracts and fermentation products have been used in the past in cosmetics and the like, but use as a topical skin agent has also been reported for *Althaea.* For example, Patent Document 1 indicates that a 1,3-butylene glycol extract of *Althaea* has tyrosinase-inhibiting activity and is used as an ingredient blended in a whitening cosmetic.

### Related Art Documents

### Patent Documents

Patent Document 1 Japanese Laid-Open Patent Application No. 60-104005

### DISLOSURE OF THE INVENTION

### Problems the Invention is Intended to Solve

However, conventional methods have been unable to adequately elicit activity by *Althaea.*

It is accordingly an object of the present invention to provide an Althaea-derived ingredient modified to increase activity. Another object is to provide a novel topical skin agent thereby.

### Means for Solving the Aforementioned Problems

The inventors perfected the invention upon conducting thoroughgoing studies to attain the aforestated object.

The present invention according to a first aspect provides a fermentation product having *Althaea* or a processed product thereof as a starting material, said fermentation product being obtained using one or more selected from microorganisms belonging to lactobacilli, microorganisms belonging to the genus Lactococcus, microorganisms belonging to the genus Leuconostoc, and microorganisms belonging to the genus Pediococcus.

In the fermentation product according to the present invention, when the microorganisms belong to lactobacilli, said microorganisms are preferably one or more selected from those of the genus Lactiplantibacillus, those of the genus Lacticaseibacillus, and those of the genus Liquorilactobacillus.

In the fermentation product according to the present invention, the microorganisms are preferably one or more selected from the group consisting of *Lactiplantibacillus plantarum*, *Lactiplantibacillus pentosus*, *Lacticaseibacillus zeae*, *Liquorilactobacillus mali*, *Lacticaseibacillus paracasei*, *Lactiplantibacillus fabifermentans*, *Liquorilactobacillus hordei*, *Lactococcus lactis*, *Leuconostoc pseudomesenteroides*, *Leuconostoc mesenteroides*, *Pediococcus acidilactici*, and *Pediococcus pentosaceus.*

The present invention according to a second aspect provides a topical skin agent containing the abovementioned fermentation product.

In the topical skin agent according to the present invention, the topical skin agent is preferably used to cause a whitening effect.

In the topical skin agent according to the present invention, the fermentation product preferably has a melanin-production-suppressing action.

In the topical skin agent according to the present invention, the fermentation product preferably has a tyrosinase-inhibiting action.

### Effect of the Invention

The present invention has exceptional activity in terms of causing a whitening effect such as melanin-production-suppressing activity, tyrosinase-inhibiting activity, or the like due to being a fermentation product having *Althaea* or a processed product thereof as a starting material and being treated by specific microorganisms. Therefore, a novel topical skin agent can be provided thereby.

### MODE FOR CARRYING OUT THE INVENTION

In the present description, "*Althaea*" is synonymous with the plant commonly understood by those skilled in the art and specifically includes in the meaning *Althaea officinalis* of the genus Althaea, family Malvaceae (scientific name: *Althaea officinalis*, also referred to by the common names "common marshmallow," "marshmallow plant," "marsh mallow," and the like). *Althaea* is a perennial plant native to Europe that grows in the wild on the coast of the Mediterranean Sea, is also widely cultivated as an herb and for herbal medicines, and is easily available.

In the present invention, microorganisms are allowed to act on *Althaea* or a processed product thereof, and a fermentation product is produced by the microorganisms. Examples of plant portions of *Althaea* on which the microorganisms act include the leaves, roots, flowers, stems, aerial sections, whole plant, or a mixture of these. The leaves, aerial sections, roots, or a mixture of these, etc., are preferred, and the leaves, roots, or a mixture of these are more preferred. The form of the plant on which the microorganisms act is a crushed product of the bulk plant or a dried product thereof, a juice, an extract, or a mixture of these (referred to as a processed product), etc., but no particular limitations are placed thereon. A juice, extract, or a mixture of these, etc., is preferred, and an extract is more preferred.

Examples of the microorganisms that act on the *Althaea* or processed product thereof include microorganisms belonging to lactobacilli, microorganisms belonging to the genus Lactococcus, microorganisms belonging to the genus Leuconostoc, and microorganisms belonging to the genus Pediococcus. More specifically, examples of the microorganisms include *Lactiplantibacillus plantarum*, *Lactiplantibacillus pentosus*, *Lacticaseibacillus zeae*, *Liquorilactobacillus mali*, *Lacticaseibacillus paracasei*, *Lactiplantibacillus fabifermentans*, *Liquorilactobacillus hordei*, *Lactococcus lactis*, *Leuconostoc pseudomesenteroides*, *Leuconostoc mesenteroides*, *Pediococcus acidilactici*, and *Pediococcus pentosaceus.* With these microorganisms, fermentation of *Althaea* is promoted well, and the activity for causing a whitening effect is exceptional. However, this does not mean that the microorganisms that can be used in the present invention are limited to these species. One type of microorganism may be used alone in treatment with the above starting material, or two or more types may be used in combination in treatment with the above starting material. Specifically, treatment by two or more different types of microorganisms may be carried out sequentially, or treatment may be carried out using two or more different types of microorganisms in combination simultaneously. Alternatively, these treatments may be combined.

As indicated in the academic journal published in April 2020 listed below, the genus of lactic acid bacteria that traditionally belonged to the genus Lactobacillus has been recategorized, and the genus name has changed for some species.

### (Recategorization of lactic acid bacteria)

- Zheng, et al. "A taxonomic note on the genus Lactobacillus: Description of 23 novel genera, emended description of the genus Lactobacillus Beijerinck 1901, and union of Lactobacillaceae and Leuconostocaceae." Int. J. Syst. Evol. Microbiol., 2020 Apr; 70(4): 2782-2858 DOI 10.1099/ijsem.0.004107

Accordingly, in the present description, microorganisms are indicated using an expression of the new taxonomy following recategorization. Microorganisms that belonged to the genus Lactobacillus under the old taxonomy and are newly referred to using the expression of the new taxonomy are collectively referred to as "microorganisms belonging to lactobacilli" and are included among the microorganisms that can be used in the present invention.

Additionally, as indicated in the academic journal published in 2000 listed below, due to the historical problem of taxonomy and nomenclature for lactic acid bacteria, numerous lactic acid bacteria that had been referred to as *Lactobacillus casei* have traditionally been referred to as *Lactobacillus paracasei* since a given point in time.

### (Synonym for old name "Lactobacillus paracasei")

- SUZUKI, Kenichiro. "Nomenclature of bacteria based on the bacteriological code and the implications in the taxonomy of lactic acid bacteria." Japanese Journal of Lactic Acid Bacteria, 2000, vol. 11, no. 2, pp. 49-59.

Accordingly, in the present description, lactic acid bacteria that have traditionally been referred to as *Lactobacillus paracasei* traditionally include those referred to as *Lactobacillus casei* as a synonym thereof.

For example, the old names for *Lactiplantibacillus plantarum*, *Lactiplantibacillus pentosus*, *Lacticaseibacillus zeae*, *Liquorilactobacillus mali*, *Lacticaseibacillus paracasei*, *Lactiplantibacillus fabifermentans*, and *Liquorilactobacillus hordei* are *Lactobacillus plantarum*, *Lactobacillus pentosus*, *Lactobacillus zeae*, *Lactobacillus mali*, *Lactobacillus paracasei*, *Lactobacillus fabifermentans,* and *Lactobacillus hordei*, respectively. All of these are microorganisms belonging to lactobacilli and are included among the microorganisms that can be used in the present invention.

The conditions under which the microorganisms act on the *Althaea* or processed product thereof are not particularly limited, provided that some type of change is imparted to components of *Althaea* by the microorganisms. However, growth conditions under which the microorganisms that act on the *Althaea* or processed product thereof proliferate to, e.g., 2-10,000 times the starting cell count, typically 5-1,000 times the starting cell count, and more typically 10-1,000 times the starting cell count, are preferred. When growth is poor, fermentation does not progress well.

Examples of auxiliary materials for growth of the microorganisms when the microorganisms act on the *Althaea* or processed product thereof include: glucose, fructose, sucrose, oligosaccharides, and other sugars; alanine, arginine, tryptophan, cysteine, and other amino acids; casein hydrolysates, protein hydrolysates, and other peptides; yeast extract, meat extract, soybean extract, and other extracts; polyoxyethylene sorbitan oleate and other surfactants with fatty acids in the side chain; and, for example, Lactobacillus MRS Broth (Difco), which is a standard medium composition for lactic acid bacteria.

However, if other components remain in addition to the Althaea-derived components, the quality of the resultant fermentation product is sometimes affected in terms of antiseptic properties, feel on use, and the like. Therefore, it is not desirable to blend more components other than the Althaea-derived components than necessary during fermentation. Consequently, when an auxiliary material is used, the proportion of the auxiliary material relative to 100 parts by mass of the *Althaea* or processed product thereof used as a starting material in fermentation is, e.g., preferably 0.001 parts by mass or greater and 5.0 parts by mass or less, and more preferably 0.01 parts by mass or greater and 0.5 parts by mass or less. However, the *Althaea* or processed product thereof may be subjected to treatment by the microorganisms without adding a starting material not derived from *Althaea.*

Discretionary, non-limiting embodiments for obtaining a fermentation product according to the present invention are explained more specifically below.

As the starting material on which the microorganisms act, for example, an extract of *Althaea* can be obtained and used. In this case, for example, a 10- to 200-fold amount of water or hot water (for example, reverse-osmosis-treated water, ion-exchanged water, tap water, well water, distilled water, ultrapure water, or the like may be used) may be added to a dried powder of the plant, and the resultant mixture may be heated to obtain a hot-water extract. The hot-water extract may be used unmodified as the starting material in the form of an extract suspension; alternatively, the water may be evaporated off to effect concentration of the extract to use as the starting material, or solids may be removed using a solid-liquid separation means such as filter filtration or centrifugation to obtain a supernatant to use as the starting material. Such a starting material may be inoculated with the microorganisms in a suitable initial concentration so that the microorganisms act on the starting material.

Treatment by the microorganisms can be carried out using methods conforming to normal aeration and static culture. In this case, the starting cell count concentration is preferably 1 × 10⁴ CFU/mL or higher and 5 × 10⁷ CFU/mL or lower, more preferably 1 × 10⁵ CFU/mL or higher and 1 × 10⁷ CFU/mL or lower. The temperature is 20-40°C, more preferably 25-37°C. The treatment time is 12 hours to 10 days, more preferably 1-3 days. Performing a treatment under such conditions, for example, can cause the microorganisms to proliferate to 2-10,000 times the starting cell count, typically 5-1,000 times the starting cell count, more typically 10-1,000 times the starting cell count, making it possible to obtain the fermentation product. After treatment by the microorganisms, the fermentation product according to the present invention may be used directly, including the microorganisms used, but it is preferable in terms of the antiseptic properties, feel on use, and the like mentioned above to remove the cells of the microorganisms used in fermentation by a solid-liquid separation means such as filter filtration or centrifugation and use the resultant supernatant as the fermentation product. It is also possible, after treatment by the microorganisms, to add various solvents to the processed product to prepare an extract or a diluted product and use the resultant extract or diluted product as the fermentation product of the present invention. Examples of solvents used in this instance include, but are not limited to, solvents commonly used in cosmetics, such as: water; ethanol, propanol, and other lower alcohols; cetyl alcohol, stearyl alcohol, and other higher alcohols; and 1,3-butylene glycol, 1,3-propanediol, glycerin, and other polyhydric alcohols. The solvents can be used individually or in mixtures of two or more.

The fermentation product according to the present invention may be used without modification as a topical skin agent or may be used by being blended in a topical skin agent production process. Specifically, the fermentation product can be used suitably, for example, as a cosmetic in the form of an emulsion, cream, cleanser, rub, sunscreen, foundation, cream foundation, or the like, or as a raw material thereof. The term cosmetic as used here includes pharmaceuticals, quasi-drugs, and cosmetics defined by the Act on Securing Quality, Efficacy, and Safety of Products Including Pharmaceuticals and Medical Devices.

Also, the form of a topical skin agent may be a pack, mask, gel, or the like in which a component that acts on the skin is carried by a suitable base material; i.e., such an embodiment may be suitably exemplified to be a component that acts on the skin in such a topical skin agent.

In discretionary, non-limiting embodiments of the present invention, the topical skin agent may be used to cause a whitening effect or may be a product that indicates functionalities such as having a melanin-production-suppressing action, having a tyrosinase-inhibiting action, or the like.

### Examples

The present invention is explained concretely below through examples, but these examples do not limit the scope of the present invention.

### (Preparation of Althaea extract)

### 1-1. Plant

Commercial dried leaves or roots of *Althaea* (scientific name: *Althaea officinallis*) were used in testing.

### 1-2. Extraction

*Althaea* extract for preculture was prepared as follows. Specifically, water (reverse-osmosis-treated water, referred to hereinafter as "RO water") was added so that the plant powder:water ratio was 1:20 (mass). Glucose was added to yield a final concentration of 0.2 w/v% and yeast extract (Difco) was added to yield a final concentration of 0.1 w/v%. The resultant combination was vigorously stirred to prepare a suspension. The suspension was aliquoted in 3-mL portions into test tubes, which were then sealed using aluminum caps, and autoclaved for 15 minutes at 121°C to obtain a hot-water extract.

Two types of *Althaea* extract for main culture were prepared: (1) no other ingredients added, and (2) 0.2 w/v% glucose and 0.1 w/v% yeast extract added. Specifically, RO water was added so that the plant powder:water ratio was 1:20 (mass). For type (1), no other ingredients were added, while for type (2), glucose was added to yield a final concentration of 0.2 w/v% and yeast extract (Difco) was added to yield a final concentration of 0.1 w/v%. The resultant combinations were vigorously stirred to prepare suspensions. The suspensions were aliquoted in 10-mL portions into test tubes, which were sealed using silicone stoppers, and autoclaved for 100 minutes at 98°C to obtain a hot-water extract.

### (2. Lactic acid bacteria)

Table 1 shows the 13 types of lactic acid bacteria used. -80°C DMSO preserved strains were inoculated onto Lactobacilli MRS Broth (Difco). After culturing for 20 hours at the optimal culture temperature, cells passaged for another generation under the same conditions were formed into a bacterial solution and provided for preculture of *Althaea* extract. The 13 types of strains used can be obtained from the public depositary institutions listed in table 1. Among those, a type of strain representing the genus and species of each of the lactic acid bacteria was obtained and used for 11 of the lactic acid bacteria used (other than Nos. 5 and 10).

**[Table 1]**

| Lactic acid bacteria No. | T: Type strain*¹ | Genus and species | ATCC*² No. | JCM*³ No. | NBRC*⁴ No. | DSM*⁵ No. | FERM*⁶ No. | Optimal culture temperature (°C) |
|---|---|---|---|---|---|---|---|---|
| 1 | T | *Lactiplantibacillus plantarum* subsp. *plantarum* | ATCC14917 | JCM1149 | NBRC15891 | DSM20174 | | 30 |
| 2 | T | *Lactiplantibacillus pentosus* | ATCC8041 | JCM1558 | NBRC106467 | DSM20314 | | 30 |
| 3 | T | *Lacticaseibacillus zeae* | ATCC15820 | JCM11302 | | DSM20178 | | 37 |
| 4 | T | *Liquorilactobacillus mali* | ATCC27053 | JCM1116 | NBRC102159 | DSM20444 | | 30 |
| 5 | | *Lacticaseibacillus paracasei* | | | | | FERM-BP 1366 | 37 |
| 6 | T | *Lactiplantibacillus fabifermentans* | | | | DSM21115 | | 30 |
| 7 | T | *Liquorilactobacillus hordei* | | JCM16179 | | DSM19519 | | 30 |
| 8 | T | *Lactococcus lactis* subsp. *lactis* | ATCC19435 | JCM5805 | NBRC100933 | DSM20481 | | 30 |
| 9 | T | *Leuconostoc pseudomesenteroides* | ATCC12291 | JCM9696 | | DSM20193 | | 30 |
| 10 | | *Leuconostoc mesenteroides* subsp. *mesenteroides* | ATCC14430 | | | | | 26 |
| 11 | T | *Pediococcus acidilactici* | ATCC33314 | JCM5885 | | DSM20333 | | 37 |
| 12 | T | *Pediococcus pentosaceus* | ATCC33316 | JCM5890 | NBRC107768 | DSM20336 | | 37 |
| 13 | T | *Streptococcus thermophilus* | ATCC19258 | JCM17834 | NBRC111149 | DSM20617 | | 37 |

| | | | | | | | | |
|---|---|---|---|---|---|---|---|---|
| Type strain^{*1}: type strain ATCC^{*2}: American Type Culture Collection JCM^{*3}: RIKEN BioResource Research Center, Microbe Division NBRC^{*4}: NITE Biological Resource Center DSM^{*5}: German Collection of Microorganisms and Cell Cultures FERM^{*6}: NITE International Patent Organism Depository | | | | | | | | |

### [Test example 1] (Use of Althaea leaves)

In preculture, 3 mL of *Althaea* leaf extract for preculture was inoculated with 0.5 v/v% bacterial solution (starting cell count concentration: approximately 1.9 × 10⁶ to 2.4 × 10⁷ CFU/mL) and static cultured for 48 hours at the optimal temperature under aerobic conditions.

In main culture, 10 mL of *Althaea* leaf extract for main culture was inoculated with 1 v/v% precultured culture (starting cell count concentration: approximately 0.4 × 10⁵ to 2.3 × 10⁶ CFU/mL) and static cultured for 72 hours at the optimal temperature under aerobic conditions.

After main culture, the viable cell count was confirmed. Specifically, 100 µL of an undiluted solution of the main cultured culture or a solution obtained by suitably diluting the main cultured culture using 0.1 w/v% yeast extract was seeded on Lactobacilli MRS agar plate medium using a spiral plater EDDY JET2 (IUL Instruments). After culturing for three days at the optimal temperature, the colonies formed were counted using a colony counter ProtoCOL3 (SYNBIOSIS), and the value of CFU (colony-forming units)/mL was calculated. The starting cell count before main culture was confirmed in the same manner.

The results are shown in table 2.

**[Table 2]**

| Lactic acid bacteria No. | Genus and species | Medium: *Althaea* leaf extract | | | |
|---|---|---|---|---|---|
| | | Not added | | GY added* | |
| | | Starting cell count | Viable cell count attained | Starting cell count | Viable cell count attained |
| | | CFU (× 10⁶/mL) | | CFU (× 10⁶/mL) | |
| 1 | *Lactiplantibacillus plantarum* subsp. *plantarum* | 0. 82 | 116.0 | 0. 82 | 12.2 |
| 2 | *Lactiplantibacillus pentosus* | 2.31 | 98.0 | 2.31 | 140.0 |
| 3 | *Lacticaseibacillus zeae* | 0.49 | 112.0 | 0.49 | 79.1 |
| 4 | *Liquorilactobacillus mali* | 1.61 | 215.0 | 1.61 | 208.0 |
| 5 | *Lacticaseibacillus paracasei* | 0.23 | 47 . 8 | 0.23 | 7.8 |
| 6 | *Lactiplantibacillus fabifermentans* | 0.37 | 26.7 | 0.37 | 24.0 |
| 7 | *Liquorilactobacillus hordei* | 0.61 | 47 . 8 | 0.61 | 54.7 |
| 8 | *Lactococcus lactis* subsp. *lactis* | 0.14 | 0.9 | 0.14 | 0.6 |
| 9 | *Leuconostoc pseudomesenteroides* | 0.48 | 23.5 | 0.48 | 8.1 |
| 10 | *Leuconostoc mesenteroides* subsp. *mesenteroides* | 0.65 | 3.0 | 0.65 | 9.1 |
| 11 | *Pediococcus acidilactici* | 0.11 | 7 . 8 | 0.11 | 4.5 |
| 12 | *Pediococcus pentosaceus* | 0. 04 | 7.8 | 0. 04 | 4. 4 |
| 13 | *Streptococcus thermophilus* | ND** | - | ND** | - |

| | | | | | |
|---|---|---|---|---|---|
| GY added*: 0.2 w/v% glucose + 0.1 w/v% yeast extract ND**: not detected after preculture | | | | | |

As a result, the lactic acid bacteria No. 13 (*Streptococcus thermophilus*) was a species or strain unsuited to preparation of a fermentation product using *Althaea* leaves as the starting material because few viable bacteria were detected after preculture.

With the other 12 strains of lactic acid bacteria, the viable cell count after culture was 6 × 10⁵ CFU/mL or higher, and thus increased at least six-fold over the starting cell count, due to culture in the *Althaea* leaf extract (exceptions being that lactic acid bacteria No. 8 (*Lactococcus lactis* subsp. *lactis*) increased about 4.3-fold in the case of "GY added," and lactic acid bacteria No. 10 (*Leuconostoc mesenteroides* subsp. *mesenteroides*) increased about 4.6-fold in the case of "Not added"). The effects when glucose and yeast extract were added to the *Althaea* leaf extract varied depending on the type of lactic acid bacteria, with the viable cell count tending to increase (e.g., lactic acid bacteria No. 2 (*Lactiplantibacillus pentosus*)), remaining basically the same (e.g., lactic acid bacteria No. 6 (*Lactiplantibacillus fabifermentans*)), or tending to be suppressed (e.g., lactic acid bacteria No. 9 (*Leuconostoc pseudomesenteroides*)). No uniform trend was observed. The magnitude of the effect on growth of the bacteria was not that remarkable in any case.

Accordingly, it was clarified that the 12 strains of lactic acid bacteria indicated above, i.e., lactic acid bacteria No. 1 (*Lactiplantibacillus plantarum* subsp. *plantarum*), lactic acid bacteria No. 2 (*Lactiplantibacillus pentosus*), lactic acid bacteria No. 3 (*Lacticaseibacillus zeae*), lactic acid bacteria No. 4 (*Liquorilactobacillus mali*), lactic acid bacteria No. 5 (*Lacticaseibacillus paracasei*), lactic acid bacteria No. 6 (*Lactiplantibacillus fabifermentans*), lactic acid bacteria No. 7 (*Liquorilactobacillus hordei*), lactic acid bacteria No. 8 (*Lactococcus lactis* subsp. *lactis*), lactic acid bacteria No. 9 (*Leuconostoc pseudomesenteroides*), lactic acid bacteria No. 10 (*Leuconostoc mesenteroides* subsp. *mesenteroides*), lactic acid bacteria No. 11 (*Pediococcus acidilactici*), and lactic acid bacteria No. 12 (*Pediococcus pentosaceus*), are suited to preparation of a fermentation product using *Althaea* leaves as the starting material.

### [Test example 2] (Use of Althaea roots)

In preculture, 3 mL of *Althaea* root extract for preculture was inoculated with 0.5 v/v% bacterial solution (starting cell count concentration: approximately 3.1 × 10⁶ to 2.8 × 10⁷ CFU/mL) and static cultured for 48 hours at the optimal temperature under aerobic conditions.

In main culture, 10 mL of *Althaea* root extract for main culture was inoculated with 1 v/v% precultured culture (starting cell count concentration: approximately 0.7 × 10⁵ to 3.6 × 10⁶ CFU/mL) and static cultured for 72 hours at the optimal temperature under aerobic conditions.

After main culture, the viable cell count was confirmed in the same manner as in test example 1.

The results are shown in table 3.

**[Table 3]**

| Lactic acid bacteria No. | Genus and species | Medium: *Althaea* root extract | | | |
|---|---|---|---|---|---|
| | | Not added | | GY added* | |
| | | Starting cell count | Viable cell count attained | Starting cell count | Viable cell count attained |
| | | CFU (× 10⁶/mL) | | CFU (× 10⁶/mL) | |
| 1 | *Lactiplantibacillus plantarum* subsp. *plantarum* | 1.44 | 222.0 | 1.44 | 129.0 |
| 2 | *Lactiplantibacillus pentosus* | 1.68 | 233.0 | 1.68 | 62.5 |
| 3 | *Lacticaseibacillus zeae* | 2.87 | 350.0 | 2.87 | 131.0 |
| 4 | *Liquorilactobacillus mali* | 3.66 | 210.0 | 3.66 | 363.0 |
| 5 | *Lacticaseibacillus paracasei* | 0. 09 | 12.7 | 0. 09 | 9.2 |
| 6 | *Lactiplantibacillus fabifermentans* | 0.78 | 114.0 | 0.78 | 37.4 |
| 7 | *Liquorilactobacillus hordei* | 0.61 | 67.7 | 0.61 | 47.0 |
| 8 | *Lactococcus lactis* subsp. *lactis* | 0.22 | 50.8 | 0.22 | 23.3 |
| 9 | *Leuconostoc pseudomesenteroides* | 0. 07 | 96.1 | 0. 07 | 57.4 |
| 10 | *Leuconostoc mesenteroides* subsp. *mesenteroides* | 0. 42 | 63.5 | 0. 42 | 23.2 |
| 11 | *Pediococcus acidilactici* | 0.29 | 24.5 | 0.29 | 33.2 |
| 12 | *Pediococcus pentosaceus* | 1. 06 | 67.7 | 1. 06 | 47.3 |
| 13 | *Streptococcus thermophilus* | ND** | - | ND** | - |

| | | | | | |
|---|---|---|---|---|---|
| GY added*: 0.2 w/v% glucose + 0.1 w/v% yeast extract ND**: not detected after preculture | | | | | |

As a result, the lactic acid bacteria No. 13 (*Streptococcus thermophilus*) was a species or strain unsuited to preparation of a fermentation product using *Althaea* roots as the starting material because few viable bacteria were detected after preculture.

With the other 12 strains of lactic acid bacteria, the viable cell count after culture was 1 × 10⁶ CFU/mL or higher, and thus increased at least ten-fold over the starting cell count, due to culture in the *Althaea* root extract. The effects when glucose and yeast extract were added to the *Althaea* root extract varied depending on the type of lactic acid bacteria, with the viable cell count tending to increase (e.g., lactic acid bacteria No. 4 (*Liquorilactobacillus mali*)), remaining basically the same (e.g., lactic acid bacteria No. 5 (*Lacticaseibacillus paracasei*)), or tending to be suppressed (e.g., lactic acid bacteria No. 6 (*Lactiplantibacillus fabifermentans*)). No uniform trend was observed. The magnitude of the effect on growth of the bacteria was not that remarkable in any case.

Accordingly, it was clarified that the 12 strains of lactic acid bacteria indicated above, i.e., lactic acid bacteria No. 1 (*Lactiplantibacillus plantarum* subsp. *plantarum*), lactic acid bacteria No. 2 (*Lactiplantibacillus pentosus*), lactic acid bacteria No. 3 (*Lacticaseibacillus zeae*), lactic acid bacteria No. 4 (*Liquorilactobacillus mali*), lactic acid bacteria No. 5 (*Lacticaseibacillus paracasei*)*,* lactic acid bacteria No. 6 (*Lactiplantibacillus fabifermentans*), lactic acid bacteria No. 7 (*Liquorilactobacillus hordei*), lactic acid bacteria No. 8 (*Lactococcus lactis* subsp. *lactis*), lactic acid bacteria No. 9 (*Leuconostoc pseudomesenteroides*), lactic acid bacteria No. 10 (*Leuconostoc mesenteroides* subsp. *mesenteroides*), lactic acid bacteria No. 11 (*Pediococcus acidilactici*)*,* and lactic acid bacteria No. 12 (*Pediococcus pentosaceus*)*,* are suited to preparation of a fermentation product using *Althaea* roots as the starting material.

### [Test example 3]

Lactic acid bacteria Nos. 1, 2, 4, 5, 6, 7, 10, and 11 were selected from among the 12 types of lactic acid bacteria (Nos. 1-12) that exhibited a viable cell count after culture of 5 × 10⁵ CFU/mL or higher in Test Example 1 (Use of *Althaea* leaves). Melanin-production-suppressing activity was examined with respect to cultures obtained using each of the lactic acid bacteria.

Specifically, after main culture by *Althaea* leaf extract to which no glucose or yeast extract was added had been completed, the culture was centrifuged for 10 minutes at 3000 rpm (1600 × g), and the supernatant was aseptically filtered using a 0.22-um filter to obtain a fermentation supernatant. The fermentation supernatant was stored at 4°C shielded from light, then stored frozen at -20°C until measurement.

Table 4 shows the results of pH measurement of the fermentation supernatant used or unfermented *Althaea* leaf extract and the evaporation residue concentration (mg/mL) when measured by allowing to cool in a desiccator after heating for three hours at 105°C.

**[Table 4]**

| Sample No. | Type of sample | pH | Evaporation residue concentration* (mg/mL) |
|---|---|---|---|
| 1-1 | Fermentation supernatant obtained using lactic acid bacteria No. 1 | 5.3 | 12.3 |
| 1-2 | Fermentation supernatant obtained using lactic acid bacteria No. 2 | 5.3 | 12.3 |
| 1-4 | Fermentation supernatant obtained using lactic acid bacteria No. 4 | 5.4 | 12.4 |
| 1-5 | Fermentation supernatant obtained using lactic acid bacteria No. 5 | 5.4 | 12.8 |
| 1-6 | Fermentation supernatant obtained using lactic acid bacteria No. 6 | 5.5 | 12.5 |
| 1-7 | Fermentation supernatant obtained using lactic acid bacteria No. 7 | 5.2 | 13.0 |
| 1-10 | Fermentation supernatant obtained using lactic acid bacteria No. 10 | 5.5 | 12.8 |
| 1-11 | Fermentation supernatant obtained using lactic acid bacteria No. 11 | 5.2 | 13.1 |
| 1-14 | *Althaea* leaf extract (unfermented) | 5.6 | 13.1 |

| | | | |
|---|---|---|---|
| Evaporation residue concentration*: allowed to cool in desiccator after heating for three hours at 105°C | | | |

Melanin-production-suppressing activity was measured by the test method shown below.

### (Test method)

### (1) Cells

As cells, B16 mouse melanoma cells (B16-F1) were used. Cells passaged 5-10 times, counting from the time of purchase, were used in testing.

### (2) Melanin production suppression test

A quantity of 500 µL of DMEM medium containing 5% FBS was placed in a 24-well plate, and B16 mouse melanoma cells were seeded 1.4 × 10⁴ cells/well (7.5 × 10⁴ cells/cm²), then cultured for 24 hours at 37°C under 5% CO₂. Thereafter, the medium was replaced with 1 mL of 0.5 mM theophylline-containing medium containing a test sample, and the cells were cultured for another three days.

As the test sample, a fermentation supernatant obtained using each of the lactic acid bacteria or unfermented *Althaea* leaf extract was added to the medium at 5% blend ratio (by volume), taking the undiluted sample solution as 100%. As a control, RO water was added to the medium in the same blend ratio instead of the fermentation supernatant obtained using each of the lactic acid bacteria or unfermented *Althaea* leaf extract.

### (3) Intracellular melanin production level

After culture was completed, the cells were washed twice with PBS(-) and fixed with 99.5% ethanol. After removing the ethanol by air drying, 1N NaOH was added to the wells and heated for 30 minutes at 80°C. A cell lysate obtained by dissolving the cells and melanin was obtained. After allowing to cool, the entire amount of cell lysate was transferred to a 96-well plate, and the 405 nm absorbance was measured. The intracellular melanin production level per culture well was determined from a calibration curve that used synthetic melanin, and the intracellular melanin production level relative to the control was determined using the following formula. Intracellular melanin production level (%) = (intracellular melanin production level of test sample/intracellular melanin production level of control) × 100

### (4) Intracellular protein level

The cell lysate was diluted ten-fold using ultrapure water (milli-Q water) and assayed by the BCA method (Pierce BCA Protein assay kit) using BSA as the reference protein, and the intracellular protein level relative to the control was determined using the following formula. Intracellular protein level (%) = (intracellular protein level of test sample/intracellular protein level of control) × 100

The results are summarized in table 5.

**[Table 5]**

| Sample No. | Type of sample | When 5% fermentation solution is added | | |
|---|---|---|---|---|
| | | Intracellular melanin production level | | Intracellular protein level |
| | | Taking RO water as 100% | Taking unfermented extract as 100% | Taking RO water as 100% |
| 1-1 | Fermentation supernatant obtained using lactic acid bacteria No. 1 | 62% | 77% | 91% |
| 1-2 | Fermentation supernatant obtained using lactic acid bacteria No. 2 | 66% | 82% | 92% |
| 1-4 | Fermentation supernatant obtained using lactic acid bacteria No. 4 | 64% | 80% | 89% |
| 1-5 | Fermentation supernatant obtained using lactic acid bacteria No. 5 | 60% | 79% | 98% |
| 1-6 | Fermentation supernatant obtained using lactic acid bacteria No. 6 | 65% | 79% | 91% |
| 1-7 | Fermentation supernatant obtained using lactic acid bacteria No. 7 | 69% | 85% | 92% |
| 1-10 | Fermentation supernatant obtained using lactic acid bacteria No. 10 | 70% | 86% | 84% |
| 1-11 | Fermentation supernatant obtained using lactic acid bacteria No. 11 | 70% | 86% | 86% |
| 1-14 | *Althaea* leaf extract (unfermented) | 79% | 100% | 87% |

As a result, while a more significant tendency was not observed with the fermentation supernatants obtained using each of the lactic acid bacteria than with the unfermented *Althaea* leaf extract in regard to the intracellular protein level (percentage relative to the intracellular protein level when no test sample was added), the intracellular melanin production level (percentage relative to the intracellular melanin level when no test sample was added) tended to decrease to a greater extent in the fermentation supernatants obtained using lactic acid bacteria No. 1 (*Lactiplantibacillus plantarum* subsp. *plantarum*), lactic acid bacteria No. 2 (*Lactiplantibacillus pentosus*), lactic acid bacteria No. 4 (*Liquorilactobacillus mali*)*,* lactic acid bacteria No. 5 (*Lacticaseibacillus paracasei*)*,* lactic acid bacteria No. 6 (*Lactiplantibacillus fabifermentans*), lactic acid bacteria No. 7 *(Liquorilactobacillus hordei*)*,* lactic acid bacteria No. 10 (*Leuconostoc mesenteroides* subsp. *mesenteroides*), and lactic acid bacteria No. 11 (*Pediococcus acidilactici*) than in the unfermented *Althaea* leaf extract.

Accordingly, it was clarified that, relative to the unfermented extract, the melanin-production-suppressing activity is increased to a greater extent in fermentation products of *Althaea* leaf extract obtained using the above lactic acid bacteria without causing a decrease in cell count.

### [Test example 4]

Lactic acid bacteria Nos. 3, 5, 8, 11, and 12 were selected from among the 12 types of lactic acid bacteria (Nos. 1-12) that exhibited a viable cell count of 5 × 10⁵ CFU/mL or higher after culture in Test example 1 (Use of *Althaea* leaves). Fermentation supernatants were prepared in the same manner as in Test example 3 (the fermentation supernatant used in Test example 3 was used for Nos. 5 and 11), and tyrosinase-inhibiting activity was examined.

Table 6 shows the results of pH measurement of the fermentation supernatants used or unfermented *Althaea* leaf extract and the evaporation residue concentration (mg/mL) when measured by allowing to cool in a desiccator after heating for three hours at 105°C.

**[Table 6]**

| Sample No. | Type of sample | pH | Evaporation residue concentration* (mg/mL) |
|---|---|---|---|
| 1-3 | Fermentation supernatant obtained using lactic acid bacteria No. 3 | 5.3 | 12.7 |
| 1-5 | Fermentation supernatant obtained using lactic acid bacteria No. 5 | 5.4 | 12.8 |
| 1-8 | Fermentation supernatant obtained using lactic acid bacteria No. 8 | 5.3 | 13.0 |
| 1-11 | Fermentation supernatant obtained using lactic acid bacteria No. 11 | 5.2 | 13.1 |
| 1-12 | Fermentation supernatant obtained using lactic acid bacteria No. 12 | 5.1 | 13.1 |
| 1-14 | *Althaea* leaf extract (unfermented) | 5.6 | 13.1 |

| | | | |
|---|---|---|---|
| Evaporation residue concentration*: allowed to cool in desiccator after heating for three hours at 105°C | | | |

The tyrosinase-inhibiting activity was measured using a partial modification of the method of Matsuda et al. (Matsuda H et al. Studies of cuticle drugs from natural sources. III. Inhibitory effect of Myrica rubra on melanin biosynthesis. Biol. Pharm. Bull., 18(8), 1148-1150 (1995)). Specifically, the fermentation supernatant obtained using each of the lactic acid bacteria or unfermented *Althaea* leaf extract was aliquoted in 50-µL portions (undiluted sample solution) into each well of 96-well microplates. After adding 50 µL of 300 mM phosphate buffer (pH 6.8) to each well and mixing the resultant combinations, the plates were preincubated for ten minutes at room temperature. Next, 25 µL of 270 U/mL tyrosinase solution (tyrosinase: mushroom derived, Sigma-Aldrich) and 25 µL of 0.06 w/v% 3,4-dihydroxy-L-phenylalanine (L-DOPA, FUJIFILM Wako Pure Chemical Corporation) were added to each well and the resultant combinations were mixed. After incubating for five minutes at room temperature, the 475 nm absorbance, which is the maximum absorption wavelength of dopachrome (intermediate of melanin biosynthesis), was measured. The final volume of the reaction system was 150 µL, of which the final blend ratio of fermentation supernatant (undiluted sample solution) was 50 µL/150 µL.

The tyrosinase inhibition rate was calculated using the following formula from the measured absorbance. In the formula, "control" represents a reaction system with RO water added instead of sample, and "blank" represents a reaction system with 50 mM potassium phosphate buffer added instead of tyrosinase. Tyrosinate inhibition rate (%) = {1 - [(S475 nm - SB475 nm) / (C475 nm - CB475 nm)]} × 100
- S475 ₙₘ:: sample
- SB_{475 nm}:: sample blank
- C_{475 nm}:: control
- CB_{475 nm}:: control blank

The tyrosinase reaction test was carried out three times on fermentation supernatants obtained using each of the lactic acid bacteria or unfermented *Althaea* leaf extract, and the mean and standard deviation were determined. The results are shown in table 7.

**[Table 7]**

| Sample No. | Type of sample | Tyrosinase inhibition rate | |
|---|---|---|---|
| | | Mean* | ±SD* |
| 1-3 | Fermentation supernatant obtained using lactic acid bacteria No. 3 | 28% | 1% |
| 1-5 | Fermentation supernatant obtained using lactic acid bacteria No. 5 | 27% | 8% |
| 1-8 | Fermentation supernatant obtained using lactic acid bacteria No. 8 | 33% | 7% |
| 1-11 | Fermentation supernatant obtained using lactic acid bacteria No. 11 | 39% | 3% |
| 1-12 | Fermentation supernatant obtained using lactic acid bacteria No. 12 | 38% | 3% |
| 1-14 | *Althaea* leaf extract (unfermented) | 17% | 2% |

| | | | |
|---|---|---|---|
| Mean* ±SD^{*}: n=3 | | | |

As a result, while the tyrosinase inhibition rate of the unfermented *Althaea* leaf extract was 17% (SD: ±2%), among fermentation supernatants obtained using each of the lactic acid bacteria, the inhibition rate was 28% (SD: ±1%) with lactic acid bacteria No. 3 (*Lacticaseibacillus zeae*), 27% (SD: ±8%) with lactic acid bacteria No. 5 (*Lacticaseibacillus paracasei*), 33% (SD: ±7%) with lactic acid bacteria No. 8 (*Lactococcus lactis* subsp. *lactis*), 39% (SD: ±3%) with lactic acid bacteria No. 11 (*Pediococcus acidilactici*), and 38% (SD: ±3%) with lactic acid bacteria No. 12 (*Pediococcus pentosaceus*).

Accordingly, it was clarified that the tyrosinase-inhibiting activity is increased to a greater extent in fermentation products of *Althaea* leaf extract obtained using the above lactic acid bacteria than in the unfermented extract.

### [Test example 5]

The 12 types of lactic acid bacteria (Nos. 1-12) that exhibited a viable cell count after culture of 1 × 10⁶ CFU/mL or higher in Test example 2 (Use of *Althaea* roots) were selected. Fermentation supernatants obtained using each of the lactic acid bacteria were prepared in the same manner as in Test example 3, and melanin-production-suppressing activity was examined.

Table 8 shows the results of pH measurement of the fermentation supernatant used or unfermented *Althaea* root extract and the evaporation residue concentration (mg/mL) when measured by allowing to cool in a desiccator after heating for three hours at 105°C.

**[Table 8]**

| Sample No. | Type of sample | pH | Evaporation residue concentration* (mg/mL) |
|---|---|---|---|
| 2-1 | Fermentation supernatant obtained using lactic acid bacteria No. 1 | 3. 9 | 16.0 |
| 2-2 | Fermentation supernatant obtained using lactic acid bacteria No. 2 | 3. 9 | 15.7 |
| 2-3 | Fermentation supernatant obtained using lactic acid bacteria No. 3 | 3. 9 | 16.0 |
| 2-4 | Fermentation supernatant obtained using lactic acid bacteria No. 4 | 4.5 | 16.0 |
| 2-5 | Fermentation supernatant obtained using lactic acid bacteria No. 5 | 4.9 | 17.3 |
| 2-6 | Fermentation supernatant obtained using lactic acid bacteria No. 6 | 3. 9 | 17.4 |
| 2-7 | Fermentation supernatant obtained using lactic acid bacteria No. 7 | 3.8 | 17.4 |
| 2-8 | Fermentation supernatant obtained using lactic acid bacteria No. 8 | 5.1 | 17.5 |
| 2-9 | Fermentation supernatant obtained using lactic acid bacteria No. 9 | 4.4 | 15.4 |
| 2-10 | Fermentation supernatant obtained using lactic acid bacteria No. 10 | 4.5 | 16.9 |
| 2-11 | Fermentation supernatant obtained using lactic acid bacteria No. 11 | 5.4 | 17.1 |
| 2-12 | Fermentation supernatant obtained using lactic acid bacteria No. 12 | 4.0 | 16.8 |
| 2-14 | *Althaea* root extract (unfermented) | 6.1 | 18.0 |

| | | | |
|---|---|---|---|
| Evaporation residue concentration*: allowed to cool in desiccator after heating for three hours at 105°C | | | |

The results are summarized in table 9.

**[Table 9]**

| Sample No. | Type of sample | When 10% fermentation solution is added | | |
|---|---|---|---|---|
| | | Intracellular melanin production level | | Intracellular protein level |
| | | Taking RO water as 100% | Taking unfermented extract as 100% | Taking RO water as 100% |
| 2-1 | Fermentation supernatant obtained using lactic acid bacteria No. 1 | 65% | 84% | 117% |
| 2-2 | Fermentation supernatant obtained using lactic acid bacteria No. 2 | 67% | 86% | 116% |
| 2-3 | Fermentation supernatant obtained using lactic | 59% | 74% | 110% |
| | acid bacteria No. 3 | | | |
| 2-4 | Fermentation supernatant obtained using lactic acid bacteria No. 4 | 62% | 81% | 108% |
| 2-5 | Fermentation supernatant obtained using lactic acid bacteria No. 5 | 54% | 81% | 121% |
| 2-6 | Fermentation supernatant obtained using lactic acid bacteria No. 6 | 54% | 75% | 126% |
| 2-7 | Fermentation supernatant obtained using lactic acid bacteria No. 7 | 55% | 76% | 128% |
| 2-8 | Fermentation supernatant obtained using lactic acid bacteria No. 8 | 65% | 85% | 99% |
| 2-9 | Fermentation supernatant obtained using lactic acid bacteria No. 9 | 61% | 79% | 117% |
| 2-10 | Fermentation supernatant obtained using lactic acid bacteria No. 10 | 65% | 85% | 112% |
| 2-11 | Fermentation supernatant obtained using lactic acid bacteria No. 11 | 57% | 79% | 112% |
| 2-13 | Fermentation supernatant obtained using lactic acid bacteria No. 12 | 50% | 69% | 125% |
| 2-14 | *Althaea* root extract (unfermented) | 75% | 100% | 102% |

As a result, while a more significant tendency was not observed with the fermentation supernatants obtained using each of the lactic acid bacteria than with the unfermented *Althaea* root extract in regard to the intracellular protein level (percentage relative to the intracellular protein level when no test sample was added), the intracellular melanin production level (percentage relative to the intracellular melanin level when no test sample was added) tended to decrease to a greater extent in the fermentation supernatants obtained using lactic acid bacteria No. 1 (*Lactiplantibacillus plantarum* subsp. *plantarum*), lactic acid bacteria No. 2 (*Lactiplantibacillus pentosus*), lactic acid bacteria No. 3 (*Lacticaseibacillus zeae*), lactic acid bacteria No. 4 (*Liquorilactobacillus mali*)*,* lactic acid bacteria No. 5 (*Lacticaseibacillus paracasei*)*,* lactic acid bacteria No. 6 (*Lactiplantibacillus fabifermentans*), lactic acid bacteria No. 7 *(Liquorilactobacillus hordei*)*,* lactic acid bacteria No. 8 (*Lactococcus lactis* subsp. *lactis*), lactic acid bacteria No. 9 (*Leuconostoc pseudomesenteroides*), lactic acid bacteria No. 10 (*Leuconostoc mesenteroides* subsp. *mesenteroides*), lactic acid bacteria No. 11 (*Pediococcus acidilactici*)*,* and lactic acid bacteria No. 12 (*Pediococcus pentosaceus*) than in the unfermented *Althaea* root extract.

Accordingly, it was clarified that the melanin-production-suppressing activity is increased to a greater extent in fermentation products of *Althaea* root extract obtained using the above lactic acid bacteria without causing an increase in cell toxicity than in the unfermented extract.

### [Test example 6]

Lactic acid bacteria Nos. 1, 3, 6, 11, and 12 were selected from among the 12 types of lactic acid bacteria (Nos. 1-12) that exhibited a viable cell count of 1 × 10⁶ CFU/mL or higher after culture in Test example 2 (Use of *Althaea* roots). Fermentation supernatants were prepared in the same manner as in Test example 5 (the fermentation supernatants used in Test example 5 were used), and tyrosinase-inhibiting activity was examined.

Table 10 shows the results of pH measurement of the fermentation supernatant used or unfermented *Althaea* root extract (the substances used in Test example 5 were used) and the evaporation residue concentration (mg/mL) when measured by allowing to cool in a desiccator after heating for three hours at 105°C.

**[Table 10]**

| Sample No. | Type of sample | pH | Evaporation residue concentration* (mg/mL) |
|---|---|---|---|
| 2-1 | Fermentation supernatant obtained using lactic acid bacteria No. 1 | 3. 9 | 16.0 |
| 2-3 | Fermentation supernatant obtained using lactic acid bacteria No. 3 | 3. 9 | 16.0 |
| 2-6 | Fermentation supernatant obtained using lactic acid bacteria No. 6 | 3. 9 | 17.4 |
| 2-11 | Fermentation supernatant obtained using lactic acid bacteria No. 11 | 5.4 | 17.1 |
| 2-12 | Fermentation supernatant obtained using lactic acid bacteria No. 12 | 4.0 | 16.8 |
| 2-14 | *Althaea* root extract (unfermented) | 6.1 | 18.0 |

| | | | |
|---|---|---|---|
| Evaporation residue concentration*: allowed to cool in desiccator after heating for three hours at 105°C | | | |

The results are summarized in table 11.

**[Table 11]**

| Sample No. | Type of sample | Tyrosinase inhibition rate | |
|---|---|---|---|
| | | Mean* | ±SD* |
| 2-1 | Fermentation supernatant obtained using lactic acid bacteria No. 1 | 6% | 5% |
| 2-3 | Fermentation supernatant obtained using lactic acid bacteria No. 3 | 15% | 6% |
| 2-6 | Fermentation supernatant obtained using lactic acid bacteria No. 6 | 16% | 16% |
| 2-11 | Fermentation supernatant obtained using lactic acid bacteria No. 11 | 15% | 3% |
| 2-12 | Fermentation supernatant obtained using lactic acid bacteria No. 12 | 11% | 25% |
| 2-14 | *Althaea* root extract (unfermented) | -10% | 33% |

| | | | |
|---|---|---|---|
| Mean tSD: n=3 | | | |

As a result, while the tyrosinase inhibition rate of the unfermented *Althaea* root extract was -10% (SD: ±33%), among fermentation supernatants obtained using each of the lactic acid bacteria, the inhibition rate was 6% (SD: ±5%) with lactic acid bacteria No. 1 (*Lactiplantibacillus plantarum* subsp. *plantarum*), 15% (SD: ±6%) with lactic acid bacteria No. *3* (*Lacticaseibacillus zeae*), 16% (SD: ±16%) with lactic acid bacteria No. 6 (*Lactiplantibacillus fabifermentans*), 15% (SD: ±3%) with lactic acid bacteria No. 11 (*Pediococcus acidilactici*), and 11% (SD: ±25%) with lactic acid bacteria No. 12 (*Pediococcus pentosaceus*).

Accordingly, it was clarified that the tyrosinase-inhibiting activity is increased to a greater extent in fermentation products of *Althaea* root extract obtained using the above lactic acid bacteria than in the unfermented extract.

## Claims

1. A fermentation product having *Althaea* or a processed product thereof as a starting material, said fermentation product being obtained using one or more selected from microorganisms belonging to lactobacilli, microorganisms belonging to the genus Lactococcus, microorganisms belonging to the genus Leuconostoc, and microorganisms belonging to the genus Pediococcus.

2. The fermentation product according to claim 1, wherein when the microorganisms belong to lactobacilli, said microorganisms are one or more selected from those of the genus Lactiplantibacillus, those of the genus Lacticaseibacillus, and those of the genus Liquorilactobacillus.

3. The fermentation product according to claim 1, wherein the microorganisms are one or more selected from the group consisting of *Lactiplantibacillus plantarum*, *Lactiplantibacillus pentosus*, *Lacticaseibacillus zeae*, *Liquorilactobacillus mali*, *Lacticaseibacillus paracasei*, *Lactiplantibacillus fabifermentans*, *Liquorilactobacillus hordei*, *Lactococcus lactis*, *Leuconostoc pseudomesenteroides*, *Leuconostoc mesenteroides*, *Pediococcus acidilactici*, and *Pediococcus pentosaceus.*

4. A topical skin agent containing the fermentation product according to any of claims 1 to 3.

5. The topical skin agent according to claim 4, used to cause a whitening effect.

6. The topical skin agent according to claim 4, wherein the fermentation product has a melanin-production-suppressing action.

7. The topical skin agent according to claim 4, wherein the fermentation product has a tyrosinase-inhibiting action.
